# EUROPEAN PATENT SPECIFICATION

(11) **EP 0 947 491 B1**
(45) Date of publication and mention of the grant of the patent: **12.05.2004**
(21) Application number: 99100257.7
(22) Date of filing: 08.01.1999
(51) Int. Cl.: C07C 9/22, A61K 9/107, A61F 6/04, A61M 5/32, C07C 7/04

(54) **Method for producing highly pure squalane**
Verfahren zur Herstellung von sehr reinem Squalan
Procédé pour la préparation de squalane très pur

(30) Priority: 01.04.1998 JP 10579598
(43) Date of publication of application: 06.10.1999
(73) Proprietor: Nippon Petrochemicals Company, Limited, Tokyo 100-8530 (JP)
(72) Inventor: Kaiya, Atsushi, Kawasaki-shi, Kanagawa 215-0021 (JP); Nakamura, Tomio, Kawasaki-shi, Kanagawa 211-0051 (JP); Wada, Hironaka, Sagamihara-shi, Kanagawa 229-1131 (JP)
(74) Representative: Strehl Schübel-Hopf & Partner

(56) References cited:
- FR-A- 2 260 327
- PATENT ABSTRACTS OF JAPAN vol. 097, no. 010, 31 October 1997 & JP 09 164162 A (NIPPON PETROCHEM CO LTD), 24 June 1997
- PATENT ABSTRACTS OF JAPAN vol. 097, no. 010, 31 October 1997 & JP 09 140790 A (NIPPON PETROCHEM CO LTD), 3 June 1997

## Description

### BACKGROUND OF THE INVENTION

### (1) Field of the Invention

This invention relates to a method for producing highly pure squalane from shark-liver oil, which squalane is excellent in stability and scarcely contains pristane being stimulative to the skin.

### (2) Prior Art

The squalane is represented by the following structural formula. It is excellent in the adaptivity to living bodies, good spreading property and safety in use, so that it is widely used for preparing almost all of cosmetics such as several kinds of creams, especially nutrient cream and medicated creams, milky lotion, toilet lotion, lipstick, foundation, and face powder. In addition, it is used as a fatting agent for high quality soap, and also used for producing medical and pharmaceutical preparations such as ointment, suppository and medically lubricating agent.

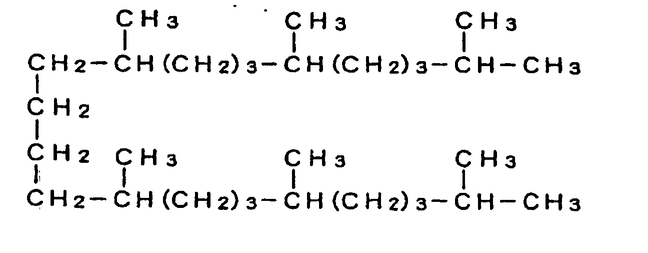

Furthermore, owing to its advantageous properties, it is used as a fiber treating agent, leather surface modifying agent, sizing agent, and face wiping cloth. Still further, in view of its good durability at low temperatures and at high temperatures, it is also used as a lubricating oil for engines and so forth.

As described above, squalane is often used for various purposes. However, it sometimes comes into question when it is brought into contact with the skin, for example, in cosmetics and medical agents, because the pristane which is contained in squalane prepared from shark-liver oil, is stimulative to the skin.

The pristane (2,6,10,14-tetramethylpentadecane) is a saturated hydrocarbon which is represented by a molecular formula of C₁₉H₄₀, and which is contained in animal oil or fat such as deep-sea shark-liver oil or else. The terms fat and oil are distinguished by their physical state. The fat is solid at ambient temperature, where the oil is liquid.

In the conventional art, the content of pristane in the commercially available squalane prepared from shark-liver oil is about 1,000 to 2,000 wt ppm.

The art to prepare squalane which does not contain pristane is disclosed in Japanese Laid-Open Patent Publication No. Hei 7-309785. This method was developed for the preparation of squalane from vegetable oils containing no pristane. Of course, this method cannot be applied to the treatment of animal oil.

The production of squalane from vegetable is small. In addition, the content of squalene as a raw material for squalane is also small, so that many processes are required for separation and concentration so as to obtain squalene. Furthermore, after the production of squalene, much waste must be discarded which causes the problem in environmental pollution.

It is considered that the anxiety to cause the disorder with the stimulation to the skin may be practically small when the quantity of pristane is so small as in the ordinary squalane prepared from shark-liver oil. However, the trend to demand a possibly safe product is increasing in recent years.

Accordingly, it is demanded to prepare squalene scarcely containing pristane from shark-liver oil as a raw material.

It is pointed out in recent years that silicone oil which is usually employed as a lubricant for condoms, is injurious to the human health. As a substitute for the silicone oil, the use of squalane is proposed in Japanese Laid-Open Patent Publication No. Hei 9-164162.

The quantity of pristane contained in the products of squalane is on the level that it causes almost no problem concerning the skin of healthy persons. However, because condoms are used in contact with the sensible mucous membrane, it is considered that a minute quantity of pristane may react upon the mucous membrane and it gives rise to undesirable influence of the lowering of sensibility for the mucous membrane. Furthermore, with some physical constitution of a user, there is anxiety that the user feels a pain when he suffers injuries in his mucous membrane.

Therefore, medical lubricating agent of squalane scarcely containing pristane is demanded.

In recent years, the symptom of arthritis caused by adjuvant composition has come into question and squalane is used for the adjuvant composition in place of liquid paraffin.

Examples of the uses of squalene or squalane for adjuvant compositions are disclosed in U.S. Patent No. 5,718,904 and European Patent 0 399 843 A2. It is disclosed in these patent gazettes that squalane emulsified with an emulsifying agent was used as adjuvant, however, the pristane in the squalane was not investigated.

In recent years, there is a tendency to keep off the application of silicone oil to injection needles because the silicone oil is difficultly excreted by metabolism. It is thus proposed to use squalane in place of the former one (Japanese Laid-Open Patent Publication No. Hei 9-140790).

However, there is no instance to give consideration to the stimulative pristane.

As described above, squalane prepared from animal oil such as shark-liver oil scarcely containing pristane is demanded. It is also required that the quantity of pristane is not more than 10 wt ppm. However, this is difficultly attained through the ordinary distillation because the decomposition or other reaction takes place.

### BRIEF SUMMARY OF THE INVENTION

It is, therefore, the primary object of the present invention to provide a method for producing highly pure squalane scarcely containing pristane which is prepared from animal oils such as shark-liver oil.

Pursuant to the above objects, the inventors have carried out extensive investigation. As a result, they found that pristane can be effectively eliminated by applying a specific treatment to squalane containing pristane, thereby accomplishing the present invention.

Therefore, a first aspect of the present invention relates to a method for producing highly pure squalane of animal oil or fat origin, wherein the content of pristane in squalane is reduced to 10 wt ppm or less by means of thin film distillation.

A second aspect of the present invention relates to a method for producing highly pure squalane of the first aspect, wherein said thin film distillation is carried out at a pressure of 0.1 mm Hg or lower and a temperature in the range of 100 to 200°C.

### DETAILED DESCRIPTION OF THE INVENTION

The present invention will be described in more detail.

The highly pure squalane is prepared by hydrogenating squalene which is obtained from shark-liver oil such as animal oil of deep-sea sharks and then subjecting the hydrogenation product to thin film distillation. The deep-sea sharks are exemplified by *Centrophorus atromarginatus* German, *Centroscymnus owstoni* German, *Deania eglantia* Jordan et Snyder, and *Centroscyllium ritteri* Jordan et Flowler.

The squalene as the raw material used in the present invention can be produced in any means from the shark-liver oil. An example of the preparation of squalene will be described.

Oil and fat content is collected from the liver of shark and the shark-liver oil is obtained by, for example, centrifugal separation. If necessary, the shark-liver oil is filtered and it is subjected to distillation, e.g. vacuum distillation in order to eliminate low temperature components and high temperature components. The thus obtained distillate (hereinafter referred to as "squalene fraction". The squalane fraction is an oily substance containing other components of esters and fatty acids.

The squalane fraction is then subjected to alkali treatment to saponify esters. After that, the reaction products are rinsed with water and dehydrated to obtain squalene scarcely containing esters and fatty acids.

In the next step, the above squalene is hydrogenated to obtain squalane.

The squalene sometimes contains unsaturated hydrocarbons which are corresponding to the pristane. When these unsaturated hydrocarbons are hydrogenated, they are converted into pristane. Even when pristane is removed from squalene before the hydrogenation, there is possibility that pristane is newly produced during the hydrogenation. Accordingly, the elimination of pristane must be done after the hydrogenation.

The catalyst used in the hydrogenation is any one of a single element, sulfide or chloride of platinum, nickel and titanium. These catalysts may be used in the forms of suspension in a reaction mixture or carrier-supported catalyst. As the catalyst carriers, well known ones such as alumina, silica-alumina, activated carbon and magnesia are used. The forms of carrier-supported catalysts are not limited, which are exemplified by any particles of pellets, granules, spheres and extruded rods. They can be selected in accordance with the conditions of hydrogenation.

The conditions for the hydrogenation are the same as those in the conventional art. That is, the temperature is in the range of an ordinary temperature to 300°C, preferably 50 to 300°C; and the hydrogen pressure is in the range of normal pressure to 200 kg/cm²G, preferably 5 to 150 kg/cm². The catalytic reaction can be carried out with a fixed bed, suspension bed, moving bed or expansion bed. Furthermore, the reaction system may be any of batch wise and continuous.

It is possible to add a solvent to squalene in the hydrogenation, which solvent is inert to the reaction and can easily be separated from squalane, such as saturated hydrocarbons of n-hexane, isooctane and so forth.

The purity of squalene in the preparation of squalane is higher than about 95 wt %, preferably higher than 98 wt %.

After the hydrogenation, the highly pure squalane is obtained from the obtained squalane by removing impurities such as pristane through thin film distillation.

The thin film distillation employed in the method of the present invention is a kind of refining method, in which a thin film of material is formed with heating under high vacuum to evaporate pristane, thereby separating pristane from the squalane to raise the purity of squalane. In this thin film distillation, the remained non-evaporated squalane is recovered with removing the pristane. So that, for the purpose to carry out the thin film distillation hereinafter referred to, a thin film distillation apparatus, thin film evaporator or the like can be employed.

The manner for forming the thin film is not limited. However, it is preferable that the thin film is forcedly formed by utilizing centrifugal force or shear force with a wiper or blade. In the method to flow down squalane by gravitation, it is necessary to take care of the difficulty in the formation of thin film. The thickness of the thin film is not especially limited, however, it is generally less than 5 mm, preferably less than 3 mm.

The thin film distillation is characterized in that the operation can be done at a low temperature to avoid the decomposition and deterioration of squalane and what is more, the treatment can be done quickly, so that the possibility of decomposition and reaction of squalane is low.

In the present invention, the quantity of pristane can be markedly reduced through the thin film distillation of squalane. It is difficult to attain such removal by means of the ordinary vacuum distillation. Meanwhile, the use of multi-stage distillation column is also difficult because the treating temperature is high. Accordingly, the thin film distillation is considered to be most effective one.

In the method of the present invention, it is possible to remove not only pristane but also other low molecular impurities through the thin film distillation. Accordingly, it is possible to attain the effect not only that pristane is eliminated from squalane but also that the purity of squalane can be improved higher than the level in conventional method.

The usable thin film distillation apparatus are exemplified by Hickman distillation apparatus, falling film evaporator, rotor-tray distillation apparatus, and brush-type molecular distillation apparatus. It is preferable that the distillation is carried out under a pressure of 0.1 mm Hg or below and a temperature of 100 to 200°C. The retention time is not especially limited, however, it is generally not longer than 30 min.

The content of pristane in the thus obtained squalane is not more than 10 wt ppm, preferably less than 5 ppm. More particularly, it is preferable that pristane is not detected substantially. If the content of pristane is more than 10 wt ppm, the quantity of pristane must be reduced to a level below 10 wt ppm by repeating the thin film distillation.

The content of pristane can be determined by the method for determining the purity of product squalane by gas chromatographic analysis according to National Formula of U.S.A.

With the method of the present invention, the purity of squalane can be improved quite easily and promptly by employing the thin film distillation.

The thus obtained squalane is quite suitable as medical, pharmaceutical and cosmetic products and as a raw material for producing them. It is especially suitable as a raw material for producing these products which are brought into contact with skin in use.

The medical and pharmaceutical products are exemplified by cooling agents for burn and scald, several kinds of creams and ointments, poultices, hair tonics, and disinfectants. The cosmetics are exemplified by several kinds of skin nutrient oils, hair ointments, milky lotions, cosmetic creams, lipsticks, cosmetic foundations, eye shadows, and bathing agents.

When squalane is used for these medical, pharmaceutical and cosmetic products, the content of pristane must be very small. In addition, the purity of squalane must be higher than 99 wt %, preferably higher than 99.5 wt %, and more preferably higher than 99.9 wt %.

### EXAMPLES

In the following passage, the present invention will be described in more detail.

### Method for Analysis

The purity of squalene and the quantity of pristane were determined according to the foregoing NF method. The conditions for the gas chromatographic analysis were as follows:
Detector: Hydrogen flame ion detector (FID)
Column: 3 mm (in. dia.) X 2 m (1)
Stationary phase carrier: Trade name: UNIPORT HP,
   80/100 mesh, made by GL Science Co., Ltd.
Carried liquid phase: Trade name: SE-30,
   3% carried, made by GL Science Co., Ltd.
Injection temp.: 350°C
Carrier gas: helium, flow rate: 60 ml/min
Hydrogen pressure: 1.0 kg/cm²
Air pressure: 1.0 kg/cm²
Feed: 2.0 µl
Preparation of sample: 2 ml of sample was dissolved into 50 ml of n-hexane

### Highly Pure Squalane

### <Example 1>

### (Step 1)

The light components and heavy components were removed from 2,000 g of deep-sea shark-liver oil (squalene content: 70.5 wt %, imported from Europe) using a stainless steel distillation column to obtain 1,380 g of squalene fraction as a main fraction of 175-180°C (0.5 mm Hg).

### (Step 2)

The squalene fraction obtained in Step 1 was saponified, which was followed by warm water rinsing. More particularly, 100 wt parts of the squalene fraction and 32 wt parts of 40 wt % sodium hydroxide aqueous solution were fed into a reaction vessel equipped with a stirrer to carry out the saponification at 85°C for 5 hours. After that, the oily phase was rinsed 5 times with warm water and it was confirmed that the oil phase was not alkaline. The obtained squalene was then warmed under reduced pressure to remove dissolved water and the treated squalene was subjected to hydrogenation. Its properties are shown in the following.

| | |
|---|---|
| Purity of squalene | 99.0 wt % |
| Content of pristane | 1500 wt ppm |

### (Step 3)

A material for hydrogenation was prepared by dissolving 10 wt parts of squalene obtained in Step 2 into 90 wt parts of n-hexane.

A tubular reactor of 1.2 cm in inner diameter and 10 cm in length was packed with 10 g of carrier-supported nickel catalyst. Hydrogenation was carried out by feeding into the reactor the n-hexane solution of squalene and hydrogen gas to obtain a squalane solution in n-hexane. Samples were taken out for 30 hours after the temperature was stabilized at 200°C.

The conditions for the hydrogenation were as follows:

| | |
|---|---|
| Flow rate of squalene solution in n-hexane | 30 ml/hr |
| Flow rate of hydrogen gas | 2.5 Nl/hr |
| Reaction temperature | 200°C |
| Pressure of hydrogen gas | 60 kg/cm²G |

### (Step 4)

Using an evaporator, n-hexane was removed from the squalane solution in n-hexane obtained in the above Step 3. Using then a centrifugal thin film distillation apparatus, light components were removed with a feed rate of 15 kg/hr and at a temperature of 150°C and a pressure of 0.03 mm Hg to obtain highly pure squalane.

The properties of the thus obtained highly pure squalane were as follows:

| | |
|---|---|
| Purity of squalane | Not lower than 99.9 wt % |
| Content of pristane | Not detected |

### <Example 2>

### (Step 1)

The light components and heavy components were removed from 2,000 g of deep-sea shark-liver oil (squalene content: 71.0 wt %, sulfur content: 24 wt ppm, obtained from Philippines) using a rotary evaporator to obtain 1,290 g of the main squalene fraction of 175-180°C (at 0.5 mm Hg).

### (Step 2)

The squalene fraction obtained in Step 1 was saponified, which was followed by warm water rinsing. More particularly, 100 wt parts of the squalene fraction and 27 wt parts of 30 wt % sodium hydroxide aqueous solution were fed into a reaction vessel equipped with a stirrer to carry out the saponification at 80°C for 6 hours with stirring.

After separating the aqueous phase from the oily phase, the oily phase was rinsed with warm water at 50°C until rinsing waste water became pH 7. The dissolved water in the obtained squalene was then removed at 75°C under a pressure of 30 mm Hg. The treated squalene was subjected to hydrogenation. Its properties are shown in the following.

| | |
|---|---|
| Purity of squalene | 99.6 wt % |
| Content of pristane | 1300 wt ppm |

### (Step 3)

A material for hydrogenation was prepared by dissolving 50 wt parts of squalene obtained in Step 2 into 50 wt parts of isooctane.

A tubular reactor of 2.0 cm in inner diameter and 10 cm in length was packed with 20 g of carrier-supported nickel catalyst. Hydrogenation was carried out by feeding into the reactor the 50 wt % squalene solution in isooctane and hydrogen gas to obtain a squalane solution in isooctane. Samples were taken out for 30 hours after the temperature was stabilized at 210°C.

The conditions for the hydrogenation were as follows:

| | |
|---|---|
| Flow rate of squalene solution in isooctane | 30 ml/hr |
| Flow rate of hydrogen gas | 5.2 Nl/hr |
| Reaction temperature | 210°C |
| Pressure of hydrogen gas | 60 kg/cm²G |

### (Step 4)

Using an evaporator, isooctane was removed from the squalane solution in isooctane obtained in the above Step 3. Using then the same centrifugal thin film distillation apparatus as the one used in Example 1, light components were removed with a feed rate of 15 kg/hr and at a temperature of 145°C and a pressure of 0.01 mm Hg to obtain highly pure squalane.

The properties of the thus obtained highly pure squalane were as follows:

| | |
|---|---|
| Purity of squalane | Not lower than 99.9 wt % |
| Content of pristane | Not detected |

### <Example 3>

The test was carried out in the like manner as in Example 1 except that the tubular reactor used in Step 3 of Example 1 was changed to another one of 2.0 cm in inner diameter and 10 cm in length and that the flow rate of squalene solution in n-hexane was 620 ml/hr and the flow rate of hydrogen gas was 10 Nl/hr.

The properties of the thus obtained highly pure squalane were as follows:

| | |
|---|---|
| Purity of squalane | 99.0 wt % |
| Content of pristane | 3 wt ppm |

### <Example 4>

The test was carried out in the like manner as in Example 2 except Step 3. In Step 3, hydrogenation was done batchwise using a 2 liter stainless steel autoclave with a stirrer. The used catalyst was made in the manner such that the catalyst used in Example 1 was finely crushed into a particle size of 150 mesh through.

The conditions for the hydrogenation were as follows:

| | |
|---|---|
| Duration of reaction | 3 hr |
| Reaction temperature | Hydrogenation was started at 130°C and, after that, the temperature was maintained at 200°C |
| Quantity of catalyst | 0.75 g |
| Quantity of squalene solution in isooctane | 1,000 g |
| Pressure of hydrogen gas | 70 kg/cm²G |

The properties of the thus obtained highly pure squalane were as follows:

| | |
|---|---|
| Purity of squalane | 99.9 wt % |
| Content of pristane | Not detected |

### <Example 5>

The test was carried out in the like manner as in Example 1 except Step 3. In Step 3, hydrogenation was done batchwise using a 2 liter stainless steel autoclave with a stirrer. The used catalyst was made in the manner such that the catalyst used in Example 1 was finely crushed into a particle size of 150 mesh through.

The conditions for the hydrogenation were as follows:

| | |
|---|---|
| Duration of reaction | 3 hr |
| Reaction temperature | Hydrogenation was started at 130°C and, after that, the temperature was maintained at 200°C |
| Quantity of catalyst | 1.5 g |
| Quantity of squalene | 200 g (Solvent was not used) |
| Pressure of hydrogen gas | 70 kg/cm²G |

The properties of the thus obtained highly pure squalane were as follows:

| | |
|---|---|
| Purity of squalane | 99.9 wt % |
| Content of pristane | Not detected |

### <Comparative Example 1>

Test was carried out in the like manner as in Example 1 except that the thin film distillation was not done.

The properties of the thus obtained squalane were as follows:

| | |
|---|---|
| Purity of squalane | Not lower than 98.5 wt % |
| Content of pristane | 1,400 wt ppm |

### <Example 6>

Squalane was prepared from commercially available shark-liver oil (purity of squalane: 99.6 wt %, quantity of pristane: 150 wt ppm). This squalane was subjected to vacuum distillation and light components were then removed by thin film distillation at 150°C and 0.05 mm Hg.

The properties of the thus obtained squalane were as follows:

| | |
|---|---|
| Purity of squalane | 99.9 wt % |
| Content of pristane | Not detected |

As described above, according to the present invention, it is possible to prepare squalane from animal oils or fats such as shark-liver oil, which squalane is not stimulative to the skin and of less deterioration by light rays or heat because the content of pristane is quite small.

Accordingly, the squalane proposed in the present invention is advantageously used as raw materials for preparing cosmetics, medical and pharmaceutical products, household utensils, food additives, fiber treating agents, lubricating agents, heating or cooling medium, coating agents, anti-corrosive agent, and so forth.

Furthermore, because the quantity of impurities is not so large which is different from the conventional squalane, the problem in the reaction between impurities and other additional components can be disregarded.

## Claims

1. A method for producing highly pure squalane of animal oil or fat origin, wherein the content of pristane in squalane is reduced to 10 wt ppm or less by means of thin film distillation.

2. The method for producing highly pure squalane as claimed in claim 1, wherein said thin film distillation is carried out at a pressure of 0.1 mm Hg or lower and a temperature in the range of 100 to 200°C.

## Patentansprüche

1. Verfahren zur Herstellung von hochreinem Squalan aus tierischem Öl oder einer Fettquelle, wobei der Gehalt von Pristan in Squalan mittels Dünnschichtdestillation auf 10 Gew.-ppm oder weniger vermindert ist.

2. Verfahren zur Herstellung von hochreinem Squalan gemäß Anspruch 1, wobei die Dünnschichtdestillation bei einem Druck von 0,1 mm Hg oder niedriger und einer Temperatur im Bereich von 100 bis 200°C durchgeführt wird.

## Revendications

1. Procédé de préparation de squalane très pur provenant d'une huile ou d'une graisse animale, dans lequel on on réduit la teneur en pristane dans le squalane à 10 ppm en poids ou moins au moyen d'une distillation en couche mince.

2. Procédé de préparation de squalane très pur selon la revendication 1, dans lequel on effectue ladite distillation en couche mince à une pression de 0,1 mm de Hg ou moins et à une température comprise entre 100 et 200°C.
